# EUROPEAN PATENT SPECIFICATION

(11) **EP 3 824 073 B1**
(45) Date of publication and mention of the grant of the patent: **30.10.2024**
(21) Application number: 19742044.1
(22) Date of filing: 19.07.2019
(51) Int. Cl.: C12N 1/18, C12R 1/865

(54) **NEW SACCHAROMYCES CEREVISIAE STRAIN FOR USE IN BREWING**
NEUER SACCHAROMYCES CEREVISIAE STAMM ZUR VERWENDUNG BEIM BRAUEN
NOUVELLE SOUCHE DE SACCHAROMYCES CEREVISIAE POUR LE BRASSAGE

(30) Priority: 20.07.2018 EP 18184696
(43) Date of publication of application: 26.05.2021
(73) Proprietor: Pinello Danmark Holding ApS, 2680 Solrød Strand (DK)
(72) Inventor: BARGHOLZ, Pia Winther, 2680 Solrød Strand (DK); CHRISTENSEN, Claus, 5330 Munkebo (DK)
(74) Representative: AWA Denmark A/S
(86) International application number: PCT/EP2019/069539
(87) International publication number: WO 2020/016423

(56) References cited:
- WO-A2-2014/135673
- CN-A- 105 802 865
- JEAN-LUC LEGRAS ET AL: "Bread, beer and wine: Saccharomyces cerevisiae diversity reflects human history", MOLECULAR ECOLOGY., vol. 16, no. 10, 1 May 2007 (2007-05-01), GB, pages 2091 - 2102, XP055507673, ISSN: 0962-1083, DOI: 10.1111/j.1365-294X.2007.03266.x

## Description

### Technical field

This invention relates to a novel *Saccharomyces cerevisiae* yeast strain suitable for use in the brewing of lager, and to brewing methods employing said yeast strain.

### Background of invention

With an annual production exceeding 1.97 billion hectoliters a year, beer is the most-produced fermented beverage in the world (Mertens S et al. Appl Environ Microbiol 81:8202-8214). The vast majority of currently produced beer is classified as either ale or lager beer, each type being produced by a unique fermentation process. Specifically, ale beer production uses the common brewer's yeast *Saccharomyces cerevisiae* and relatively high fermentation temperatures (typically 18 to 25°C). In contrast, lager beer (with Pilsner beer as the most popular and commonly known type of lager beer) is fermented at lower temperatures (5 to 15°C), followed by a period of cold storage (lagering), which is a traditional practice vital for the beer's characteristically clean flavor and aroma. Lagers are not fermented by *S*. *cerevisiae* but by the lager yeast strain *Saccharomyces pastorianus* (formerly known as *Saccharomyces carlsbergensis*)*,* which is a hybrid yeast that combines the desirable fermentation characteristics of *S*. *cerevisiae* with the cold tolerance of its other parent, *S*. *eubayanus* (Mertens S, supra). The hybridisation event happened approximately 500-600 years ago and therefore *S*. *pastorianus* may be considered as a newly evolving species. The happenstance of the hybridisation event created a novel species, with unique genetic characteristics. Lager yeast strains retain the chromosomes of both parental species and also have sets of novel hybrid chromosomes that arose by recombination between the homeologous parental chromosomes.

The lager yeasts are subdivided into two groups (Group 1, "Saaz/Carlsberg" and Group 2, "Frohberg") based on the *S*. *cerevisiae : S. eubayanus* gene content and the types and numbers of hybrid chromosomes, and further on the geographic heritage of the strains (C Monerawela et al. Biotechnology Advances, Vol. 35, Issue 4, July 2017, p.512-519). The division was supported by molecular analyses of transposon distribution in these strains (Mertens S, *supra*)*.* Only recently, the differences in fermentation performance of these two groups were analyzed. It was shown that group I/Saaz yeasts are better adapted to low-temperature growth conditions (10°C), while group II/Frohberg yeasts ferment better at a higher temperature (22°C). Differences in sugar utilization became apparent, as group II yeasts utilize maltotriose and group I yeasts do not. Additionally, flavor differences were identified showing that Saaz strains produce severalfold-lower levels of, e.g., isoamyl acetate (banana flavor) than Frohberg strains (Mertens S, *supra*)*.*

In contrast to wine and ale beer yeasts, the diversity of lager yeast strains have remained very restricted since no new lager yeast strains have been isolated from the wild since the hybridisation event which created *S*. *pastorianus* 500-600 years ago (J Wendland, Eukaryotic Cell p. 1256-1265, 2014). This limited genetic variation may explain the relatively limited aromatic diversity between the available lager beer yeasts compared to, for example, wine and ale beer yeasts.

Following the discovery of *S*. *eubayanus* being one of the parental species of *S*. *pastorianus,* research has been conducted worldwide at creating new hybrids in the laboratory, but there still remains a need for new natural, or wild lager yeasts, both with a view to providing lager beers with improved body, mouthfeel and texture, and with a capability to produce lagers of different alcoholic strengths, for which there is an increasing demand.

### Description of drawings

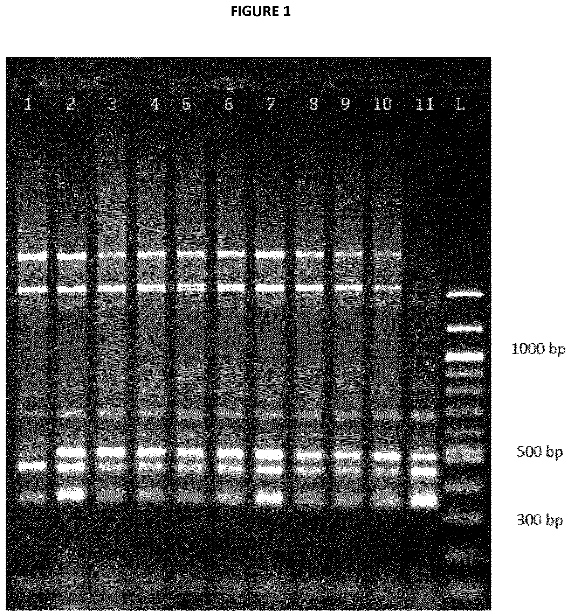

**Figure 1** shows a photograph of the PCR fingerprint agarose gel electrophoresis of the new low-temperature fermenting yeast strain NCYC 4260 performed by NCYC. The PCR (polymerase chain reaction) fingerprinting technique uses primers which are designed to anneal to the Long Terminal Repeat (LTR) section of the extensively described **Ty** (transposon yeast) retrotransposon family. Retrotransposons are naturally occurring mobile genetic elements which can "jump" into and out of genes in all organisms, including yeast. When they jump "out" they leave a footprint in the DNA that the primers can bind to. As the insertions are random, different strains of yeast will have their footprints in different places in the genome. PCR amplifies between these primers, which stick to the footprints, and creates pieces of DNA fragments which reflect the distance between these footprints in the individual strain. Agarose gel electrophoresis is used to separate the fragments and visualise them, leading to characteristic "fingerprints" (patterns of bands) for each strain. In order for two colonies to have the same fingerprint they must be genetically identical, so the PCR fingerprint is unique.

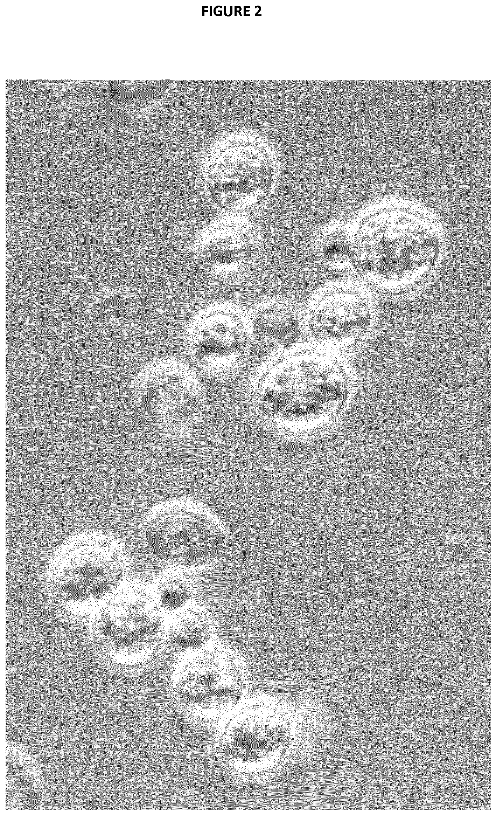

**Figure 2** shows a microphotograph of some yeast cells from the yeast strain deposited as NCYC 4260. Magnification app 1000x.

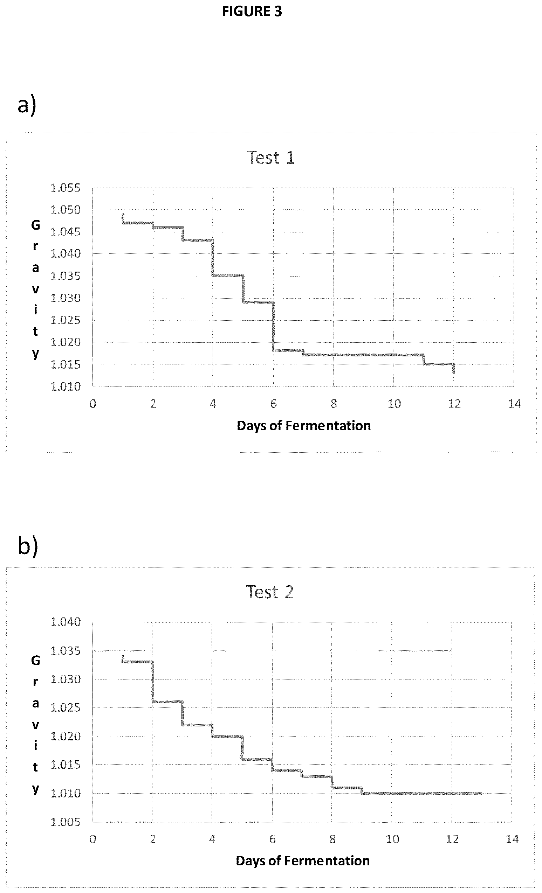

**Figure 3** shows the fermentation profile of NCYC 4260 fermenting two different original gravity worts: a) **Test 1,** 1.055 gr/ml (13.5 °P) and b) **Test 2,** 1.035 gr/ml (9.0 °P). As can be seen, fermentation first flattens out after 8-10 days with the NCYC 4260 strain, and is still active up to about 12 days.

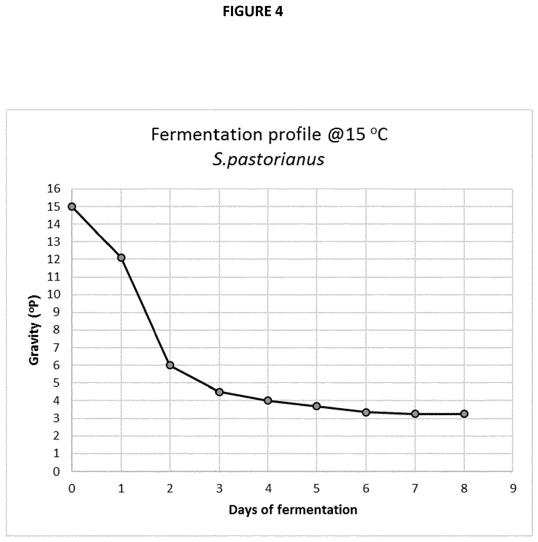

**Figure 4** shows the fermentation profile of an industrial lager brewing yeast (*S.pastorianus*)*.* fermenting a 15 °P (= original gravity 1.061 gr/ml) wort. Fermentations were carried out at 15 °C. As can be seen, fermentation flattens out after 6-7 days with *S.pastorianus* (adapted from J. Ekberg et al. FEMS Yeast Res 13 (2013) 335-349).

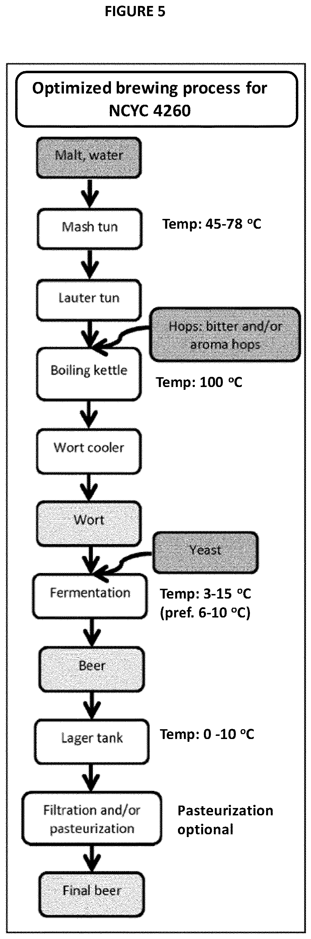

**Figure 5** illustrates an optimized beer brewing process for the new low-temperature fermenting yeast strain NCYC 4260.

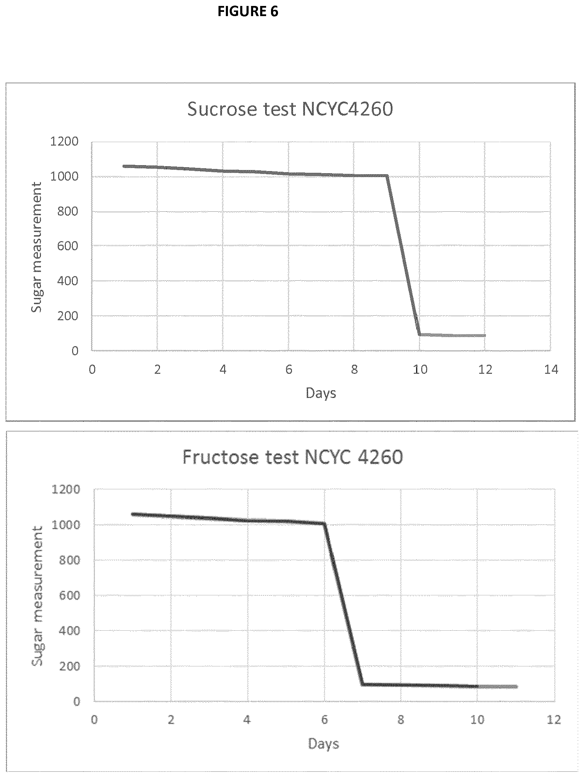

**Figure 6** shows the fermentation profile of NCYC 4260 fermenting two different sugars: Sucrose (top) and Fructose (bottom). As can be seen, NCYC 4260 efficiently ferments both sugars to very low final gravity (FG) values. For Sucrose the OG of 1060 is fermented to an FG of 870 with an alcohol content of 9.92 % vol/vol and an attenuation of app. 78%; for Fructose the OG of 1061 is fermented to an FG of 860 with an alcohol content of 10.19 % vol/vol and an attenuation of app. 77%.

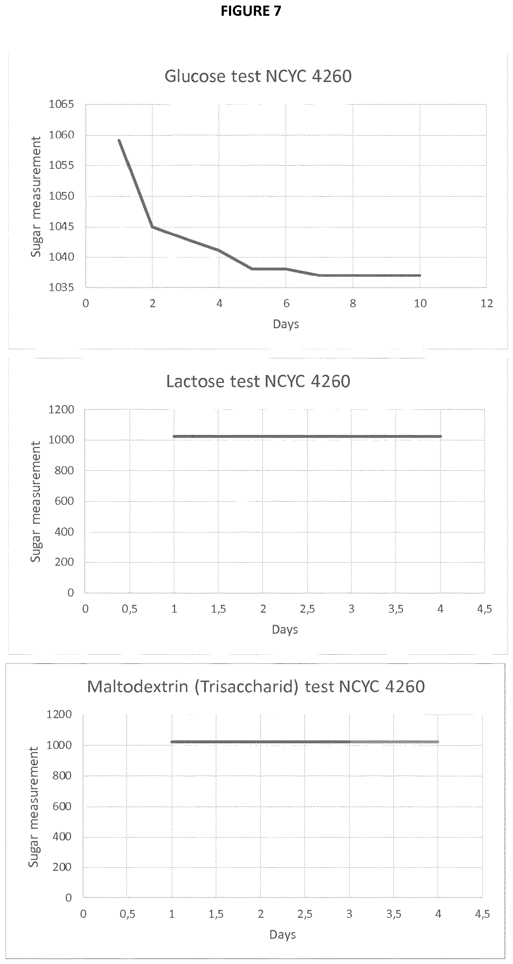

**Figure 7** shows the fermentation profile of NCYC 4260 fermenting three different sugars: Glucose (top), Lactose (middle) and Maltodextrin (bottom). As can be seen, NCYC 4260 is incapable of fermenting Lactose (a disaccharide) and Maltodextrin (a trisaccharide), and only inefficiently ferments pure glucose.

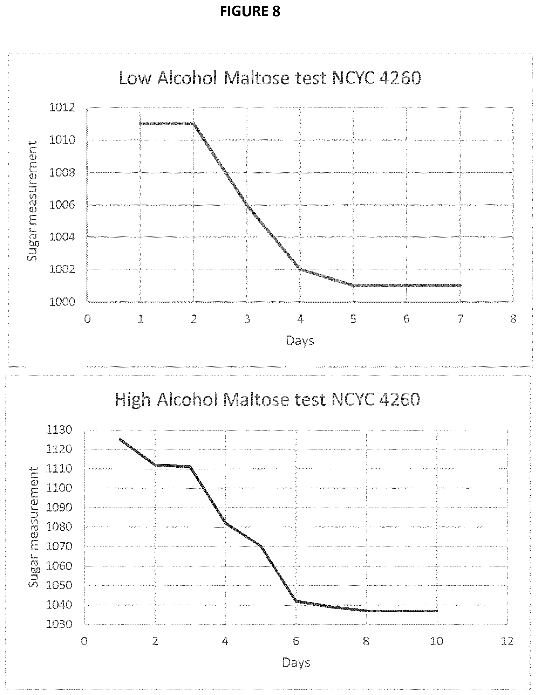

**Figure 8** shows the fermentation profile of NCYC 4260 fermenting Maltose at two different concentrations: Low (original gravity = 1011, top) and High (original gravity =1125, bottom). At low OG conditions the final gravity (FG) was 1001 with an alcohol content of 1.33 % vol/vol and an attenuation of 90.9%. At high OG conditions the final gravity (FG) was 1037 with an alcohol content of 11.96 % vol/vol and an attenuation of 70.4%.

As can be seen, NCYC 4260 efficiently ferments maltose under both sets of conditions, but that under low OG conditions the attenuation is much higher, possibly because of the lower alcohol content. This means that an even lower alcohol content can be achieved by starting the brew with an OG lower than 1011. The figure also shows that a beer of approximately 12% vol/vol (and possibly even higher by adding easier fermentable sugars to the brew) is achievable using NCYC 4260.

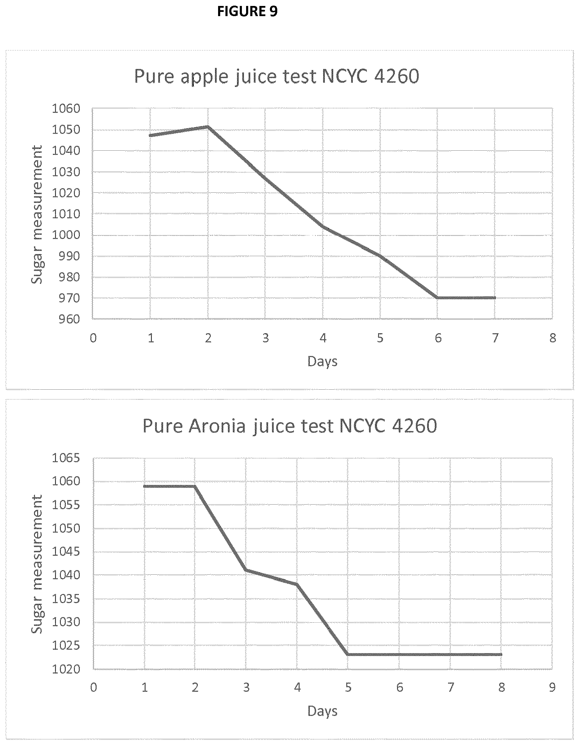

**Figure 9** shows the fermentation profile of NCYC 4260 fermenting pure Apple juice (top) and pure Aronia juice (bottom). As can be seen, NCYC 4260 efficiently ferments pure Apple juice to a very low FG (979), at which level an alcohol content of >10% vol/vol was achieved. Aronia juice could be fermented to an an alcohol content of 4.8% vol/vol and an FG of 1023.

### Description of the invention

As mentioned hereinabove, the commercial go-to lager beer yeast strain *S*. *pastorianus* was created in a hybridisation event which happened approximately 500-600 years ago. To the best of the inventors' knowledge, no new lager (i.e. low temperature fermenting) yeast has ever been isolated from the wild in Europe. The lager yeasts used today are predominantly, if not exclusively, produced by careful selection and laboratory breeding strategies.

The present novel organism was isolated by the inventors from a reed growing in a nature reserve at the seaside south of Copenhagen, Denmark. The expectation was to find a high temperature fermenting ale-yeast, but experiments with the newly found yeast culture revealed surprisingly that it was capable of fermenting at low temperatures normally associated with lager-yeasts.

The organism was isolated (see Example 1) and biologically pure cultures thereof were produced by techniques considered standard by those skilled in the art, and subsequently analyzed by two independent laboratories: Escarpment Laboratories, Guelph, Ontario in Canada, and by the Budapest depository institution National Collection of Yeast Cultures (termed "NCYC" herein), Food Research Institute, Norwich, Norfolk, England, and was found to be a single strain organism (Example 1 and figure 1) belonging to the species *Saccharomyces cerevisiae.* The organism was filed for patent purposes according to the procedures established by the Budapest Treaty, at the NCYC on 18/6/2018. The accession number of the organism in this repository is **NCYC 4260.**

Analyses of the strain are ongoing, and it has been established with a high degree of certainty that the strain is a *Saccharomyces cerevisiae,* possibly a hybrid hereof with *S.uvarum* or *S.paradoxus.* It may thus be a hitherto unknown bottom-cropping *S*. *cerevisiae* or a new cold resistant hybrid.

In a **first aspect** the present invention therefore provides a yeast strain belonging to the species *Saccharomyces cerevisiae* having the identification code NCYC 4260.

In a particular embodiment there is further provided a biologically pure culture of a yeast strain deposited as NCYC 4260 or subcultures thereof. In another embodiment there is also provided a biologically pure culture of a yeast having all the identifying characteristics of the yeast strain deposited as NCYC 4260 or subcultures thereof. In further embodiments a yeast cell obtained by a cultivation process of a yeast strain deposited as NCYC 4260 and a yeast cell culture comprising yeast cells from the yeast strain deposited as NCYC 4260 are provided.

In a **second aspect** the present invention provides the use of the yeast strain or yeast culture according to the first aspect for the fermentation of worts and other sources of fermentable sugars in the preparation of an alcoholic beverage, wherein the fermentation is carried out at a temperature between 3-15 °C, such as 3-10 °C or such as 6-10 °C.

In preferred embodiments there is provided the use of the yeast strain or yeast culture according to the first aspect for the fermentation of worts and other sources of fermentable sugars, optionally mixtures of sugars, wherein the resulting alcoholic beverage is beer, sake, wine, cider, mead, root-beer, ginger-beer and kampuchea.

Experiments with the new yeast strain (referred to in the following as NCYC 4260), see Example 3, have revealed that it has the capacity to produce lager with a low alcohol content (1-3% ABV), which by comparative testing (see Example 4) was found qualitatively on par with lagers of normal commercial strength (4-6% ABV) as regards typical qualitative parameters such as body, texture and mouthfeel, but surprisingly also perceived to have a higher than actual alcohol strength.

Other experiments with NCYC 4260 have revealed that the yeast tolerates an alcoholic strength of 14% ABV and at least 300 IBU (international bitter units), and it is thus reasonable to expect that beers or other alcoholic beverages such as cider of at least 10% ABV can be produced with NCYC 4260, rendering NCYC 4260 an extraordinarily versatile lager yeast. Experiments (Experiment 3d) have shown that a high-concentration maltose fermentation may lead to an alcohol content of 11,74 % vol/vol, and that apple juice may be fermented to a cider with an alcohol content > 10% vol/vol.

Further experiments with NCYC 4260 have revealed that the NCYC 4260 yeast operates at very high attenuation (>90%) at low original gravity (OG) values, and thus is capable of fermenting low-OG worts and other fermentable starting materials to final beverages having an alcohol content of between 0.1 - 0.5% vol/vol. This range is considered "non-alcoholic" or "alcohol-free" in many countries, including most European Union member states and the US.

Experiments with NCYC 4260 have in general shown that the yeast is well-suited for the production of fermented beverages. NCYC 4260 is thus not limited to the fermentation of worts derived from barley and similar sources, but may be used with other fermentable starting materials.

As an example, fermentation of pure apple juice with NCYC 4260 provided a dry, high-alcohol (>10% vol/vol) cider, see example 3f and figure 9, top. Aronia juice was also tested (figure 9, bottom), but achieved only an alcohol content of about 4.8 % vol/vol.

A test of the capability of NCYC 4260 to ferment some different sugars was carried out. Thus maltose (two starting concentrations), sucrose, fructose, glucose, lactose and maltrodextrin were tested under identical conditions. These are sugars that typically are used in the fermentation of beers, cider and wine.

From this test it could i.a. be concluded (see Experimental and figures 6-9) that NCYC 4260 surprisingly is a highly efficient fructose-fermenting yeast. Also somewhat surprisingly, NCYC 4260 is not very efficient at fermenting pure glucose (fig. 7 top), whereas the glucose-containing disaccharides sucrose (glucose-fructose disaccharide) and maltose (glucose disaccharide) were found to be excellent substrates for NCYC 4260 (fig. 6 top and fig. 8). Less surprisingly, NCYC 4260 did not ferment lactose or the trisaccharide maltodextrin (fig.7).

In a **third aspect** the present invention thus provides a method of preparing a fermented product, said method comprising
(a) contacting a source of sugar with a yeast cell from the yeast strain NCYC 4260 or a yeast culture provided therefrom, according to the first aspect,
(b) conducting a fermentation process; and
(c) obtaining the fermented product from step (b).

In an embodiment of the method of the third aspect, the fermented product is a beverage. In a further embodiment of the method of the third aspect, the fermented product is an alcoholic beverage.

In exemplary embodiments of the third aspect, the fermented product is selected from beer, sake, wine, cider, mead, root-beer, ginger-beer, cacha 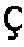 a and kampuchea.

In a preferred embodiment of the third aspect, the fermented product is lager beer obtained by the method according to third aspect and subsequently subjected to the traditional lagering and storage of such beers, which are well-known to the skilled person (see figure 5).

In a preferred embodiment of the third aspect, the fermented product is apple cider or pear perry obtained by the method according to third aspect and subsequently subjected to the traditional lagering and storage of such beverages, which are well-known to the skilled person.

The inventors have discovered that it is possible to use more than one source of sugar in the fermentations employing the yeast strain NCYC 4260. This has the advantage that either the FG, the final alcohol content, or both parameters can be fine-tuned in a given fermented product. Thus, a sugar which is only inefficiently fermented by the yeast strain NCYC 4260 may be added to the fermentation mixture which will result in a higher FG without increasing the alcohol content substantially. On the other hand, a sugar which is efficiently fermented (like sucrose or fructose) by the yeast strain NCYC 4260 may be added to the fermentation mixture which will result in an increased alcohol content. It is known from the initial screening of NCYC 4260 that the yeast survives an alcohol content of at least 14% vol/vol.

Therefore, in another embodiment of the third aspect more than one source of sugar is mixed and subsequently contacted with a yeast cell from the yeast strain NCYC 4260 or a yeast culture provided therefrom, according to the first aspect, followed by
(b) conducting a fermentation process; and
(c) obtaining the fermented product from step (b).

Comparing efficiencies reveals that NCYC 4260 has an efficiency of around 70% under normal brewing conditions (amount of yeast, concentration of fermentable sugar) whereas *S.pastorianus* and other industrial lager yeasts typically have efficiencies around 75-78% or higher. This is calculated as the fraction **(Plato_{OG}** - **Plato_{FG})/Plato_{OG}** such that a yeast fermenting a wort having an original gravity (OG) of 15 °Plato resulting in a beer with a final gravity (FG) of 3 °Plato has had an efficiency of (15-3)/15 = 80%. Similarly, a yeast having fermented a wort with an original gravity (OG) of 1050 (=1,050 gr/ml) to produce a beer with a final gravity (FG) of 1015 (=1,015 gr/ml), has had an efficiency of (1050 -1015)/(1050 -1000) = 70%.

This is by no means a disadvantage. The slower fermentation and lower attenuation by NCYC 4260 gives the beer a character which is not readily found in a faster produced lager, as witnessed by the tasting example disclosed herein.

In a particularly preferred embodiment of the present invention there is provided a method of preparing beer comprising the steps of:
a) providing a wort; and
b) fermenting the wort with the yeast strain NCYC 4260 or a yeast culture provided therefrom to obtain a beer under sterile conditions and with the yeast strain NCYC 4260 or a yeast culture provided therefrom as the only species of yeast added;and
c) optionally pasteurizing said beer obtained under b); and
d) optionally subjecting said beer to a period of cold storage (lagering).

In an embodiment the wort contains more than one source of sugar.

In a further embodiment, the fermentation of the wort with the yeast strain NCYC 4260 or a yeast culture provided therefrom is carried out for at least 12 days.

In a another embodiment the fermentation of the wort with the yeast strain NCYC 4260 or a yeast culture provided therefrom is carried out at a temperature of between 3-15 °C, such as between 3-10 °C, such as between 6-10 °C.

In a further embodiment the fermentation of the wort with the yeast strain NCYC 4260 or a yeast culture provided therefrom provides a beer with an alcohol content of between 0.05 and 0.5% (vol/vol). The final gravity (FG) may be as low as 1000, but may be higher if a non-fermentable sugar is added to the brew.

In a further embodiment the fermentation of the wort with the yeast strain NCYC 4260 or a yeast culture provided therefrom provides a beer with an alcohol content of between 0.5 and 2.8% (vol/vol) and a final gravity (FG) of between 1001 and 1025, such as 1010.

In a further embodiment the fermentation of the wort with the yeast strain NCYC 4260 or a yeast culture provided therefrom provides a beer with an alcohol content of between 2.8 and 14% (vol/vol) and a final gravity (FG) of between 1001 and 1037, such as 1010.

In a **fourth aspect** the present invention provides an alcoholic beverage such as beer which is obtainable by the fermentation process described hereinabove, using the yeast strain NCYC 4260, and optionally including a pasteurization and/or a lagering step. As witnessed by the tasting example (Example 4), the NCYC 4260 yeast culture provides a final beer produced according to this method with a body, mouthfeel and perception of alcohol strength which is on par with traditional lager beer having a higher alcohol content. These qualities of the beer are not objectively measurable.

In a preferred embodiment of the fourth aspect the alcoholic beverage such as beer obtainable by the fermentation process described hereinabove still contains live or viable yeast cells identical to yeast cells from the yeast strain deposited as NCYC 4260 or subcultures thereof. In another embodiment of the fourth aspect the alcoholic beverage such as beer obtainable by the fermentation process described hereinabove has been pasteurized or treated by other means with the intent to remove live or viable yeast cells.

### Examples

### Example 1

A sample of plant material was collected by the inventors in the wild at Solrød Strand, at a nature reserve at the seaside south of Copenhagen, Denmark. The sample of plants was put in a sterile bag containing a sterile malt-based media (prepared from 1 part malt sugar + 2 parts water which were mixed and boiled for 30 minutes, and added to a sterile closed container. The closed container was heated at 80°C for 30 min, and left to cool to room temperature before use).

After 5 days in the malt based sterile media, the plant parts were removed. The filtrate was streaked on sterile agar-agar plates and left to grow for about 5 days. A good-sized colony was selected and mixed in a malt-based media. The mixture was divided in two fermentation jars, set at 6-9 °C and 20 °C respectively, and left to ferment for about a week.

Samples of yeast from the top part of the fermentation jars were transferred to a petri dish for isolation of a single colony. A single colony was selected from each jar, and tested for about a week to survive an environment of:
- 14% alcohol
- 300 IBU (Hops added)

The yeast was viable and visibly actively fermenting under both sets of conditions even after a week. From the two testing temperatures, the colder test conditions produced by far the best result and the "brew" had a good malty smell. The brew fermented under warmer test conditions didn't smell very pleasant, and was rejected without further testing.

At this stage it was clear that the isolated yeast strain was a cold fermenting yeast capable of fermenting a malt-based beverage. Fermentation has been found to be optimal at 6-9 °C, but the yeast will still ferment at lower temperatures above 0 °C degrees and higher than 10 °C, preferably in the range of 3-15 °C.

### Example 2a PCR fingerprinting analysis

A single sample of the yeast isolated in Example 1 hereinabove, and now deposited with as NCYC 4260 was submitted for analysis at NCYC¹ in the form of a suspension of yeast cells in liquid in a microcentrifuge tube. An inoculum was taken from this sample and streaked on YM agar plates to provide sufficiently spaced colonies for analysis.

After 3 days growth at 25°C, colonies on the plates were of sufficient size for analysis. No bacteria, aberrant colony types or other contaminants were detected on the plates. The twelve tracks seen on the photograph of the electrophoresis gel are depicted in Figure 1 and are as follows:

| | | | |
|---|---|---|---|
| 1. | Confluent growth | 7. | Single colony 6 |
| 2. | Single colony 1 | 8. | Single colony 7 |
| 3. | Single colony 2 | 9. | Single colony 8 |
| 4. | Single colony 3 | 10. | Single colony 9 |
| 5. | Single colony 4 | 11. | Single colony 10 |
| 6. | Single colony 5 | 12. | 100 bp ladder |

### Results:

The pattern produced by the confluent growth of the NCYC 4260 sample matched that of all the single colonies tested. The one anomaly is that the upper band in the doublet at 500/450bp is under-represented in the confluent sample compared to the colonies. In addition, the PCR in lane 11 (colony 10) is under-represented for the higher molecular weight bands present in all the other lanes (approx. 3Kb and 1.5Kb). However it is clear from the high degree of similarity of the fingerprint patterns that this sample is composed of a single uniform strain.

### Example 2b DNA Sequence Analysis

Apart from the fingerprinting analysis mentioned in Example 1 hereinabove, NCYC 4260 was analyzed at Escarpment Labs, Canada by Sanger sequencing to differentiate this yeast from others at the genomic level.

ITS-PCR was used to amplify the internally transcribed spacer region of the fungal ribosomal DNA. These are highly variable sequences of great importance in distinguishing fungal species by PCR analysis. Sequencing of the ITS-PCR fragment using the primers ITS1 and ITS4 yields a band (400-900nt depending on species) was submitted for sequencing and BLAST'd in order to identify NCYC 4260 to the species level.

The procedure was carried out as follows:
a. Extract ribosomal DNA with Instagene Matrix
b. Perform PCR according to Pham, T. et al. (2011). Evaluation of ITS PCR and RFLP for Differentiation and Identification of Brewing Yeast and Brewery 'Wild' Yeast Contaminants. Journal of the Institute of Brewing. http://doi.org/10.1002/j.2050-0416.2011.tb00504.x
c. Check for amplification using agarose gel electrophoresis
d. Clean up DNA sample using QiaQuick DNA cleanup kit
e. Obtain sequence data.
f. QC sequence data (.ab1 files) using 4Peaks software and selecting only high quality traces (Mac; https://nucleobytes.com/4peaks/index.html)
g. BLAST search the nucleotide sequence from f) using https://blast.ncbi.nlm.nih.gov/Blast.cgi?PROGRAM=blastn&PAGE TYPE=BlastSearch &LINK LOC=blasthome

### Results:

The BLAST search of the NCYC 4260 sequence did not identify NCYC 4260 as one of the strain sequences on file with the NCBI (National Center for Biotechnology Information, USA) database. However, significant similarities with a large number of *S.cerevisiae* sequences on file with NCBI were found during the search, which strongly indicates that NCYC 4260 belongs to this species. However, the BLAST search also had some hits for hybrids, which means that NCYC 4260 could also be a *S.cerevisiae* x *S.uvarum* or *S.cerevisiae* x *S.paradoxus* hybrid.

Additionally, NCYC 4260 was analyzed by PCR at Escarpment Labs using primers SD5A and SD6B for the STA1 gene. The STA1 gene was not detected in NCYC 4260, indicating this yeast does not contain the STA1 gene and therefore is not a diastatic yeast (*S*. *cerevisiae* var. *diastaticus*)*.*

### Example 3 Brewing examples

Brewing tests using NCYC 4260 were performed to produce lagers of normal ABV (alcohol strength) 5 % (test 1) and a lager of lower strength, ABV 2,8 % (test 1). Brewing followed the general set-up outlined in Figure 5.

A propagation of the new yeast strain NCYC 4260 was made for the test brewing. A malt based media as described hereinabove was mixed in a conical flask with a yeast lock on top. The mixture was set to propagate at 8-9 °C for 4 days. The yeast was then ready to transfer to the fermentor together with the wort. The two beers were fermented at the same temperature, 9.4 °C. Brewing results (see also the fermentation profile, figure 3 a and b) show an efficiency for NCYC 4260 of about 65-75% which is lower than for a typical commercial lager yeast (Carlsberg) which is about 78-80% (figure 4).

### Example 3a

### Fermentation profile see figure 3a

Fermentation trials were carried out in 25 liter of wort (all grain test brew).

The wort was prepared with malt (4.47 kg Pilsnermalt, 0.60 kg Munich 15 and Chateau Biscuit 0.20 kg). The malt was then crushed and mixed with warm water (mash in) to reach an initial sugar content of SG 1052 (specific gravity 1.052 gr/ml) as measured with a hydrometer. It was then heated to 62.0 °C for 20 min for saccharification followed by heating to 68.0 °C for 20 min (a-Amylase activity). The last step was heated to 72.0 °C in 20 min, then the mash out at 78.0 °C for 1 min (sparge). Electric heat source of the pot was used to maintain the desired temperature during the mash.

Initial set-up: the wort was boiled for 75 minutes in total. The bittering hops Hallertauer Tradition (14.32 g) were added to the wort after 15 minutes. Then after 30 minutes, 10.86 g Hallertauer Hersbrucker was added to the wort. For the last 10 minutes, aroma hop Hallertauer Hersbrucker 3.70 g) was added together with 0.33 tsp Irish Moss (boiled last 10 minutes). After boiling, the wort was transferred to a 30L fermenter, and fresh yeast (NCYC4260) was added. Then the fermenter was closed by water locks, and kept at a temperature of 9.4 °C to start fermenting. Fermentation was carried out at 9,4 °C. After 12 days a sample was taken for measuring the FG (final gravity), which was 1014 (1.014 gr/ml).

The efficiency of the NCYC4260 yeast in the fermentation was (1052-1014)/(1052-1000)=**73%**.

### Example 3b

### Fermentation profile see figure 3b

Fermentation trials were carried out in 25 liter of wort (all grain test brew).

The wort was prepared with malt (Pilsnermalt 2.90 kg, and CHÂTEAU CARA BLOND 0.27 kg). The malt was then crushed and mixed with warm water (mash in) to reach an initial sugar content of SG 1034 as measured with a hydrometer. It was heated to 62.0 °C for 20 min for saccharification followed by heating to 68.0 °C for 20 min (a-Amylase activity). Last step was heated to 72.0 °C in 20 min, then the mash out at 78.0 °C 1 min (sparge). Electric heat source of the pot was used to maintain the desired temperature during the mash.

Initial set-up: the wort was boiled for 75 minutes in total. The bittering hops Nothon Brewer (25g) was added to the wort 15 minutes after start. Then after 30 minutes, 10 g Hallertauer Hersbrucker was added to the wort. The last 10 minutes aroma hop Hallertauer Hersbrucker (15 g) was added together with 0.33 tsp Irish Moss and 120 g of cane sugar (boiled last 10 minutes).

After boiling, the wort was transferred to a 30L fermenter, and fresh yeast (NCYC4260) was added. Then the fermenter was closed by water locks, and kept at a temperature of 9,4 °C to start fermenting. Fermentation was carried out at 9,4 °C. After 12 days a sample was taken for measuring the FG, which was 1012 (1.012 gr/ml).

The efficiency of the NCYC4260 yeast in the fermentation was (1034-1012)/(1034-1000)=**65%**

### Example 3c

### Theoretical example

Fermentation is carried out in 25 liter of wort (all grain test brew).

To prepare for a high gravity brew it is important to check the equipment. In order to brew a strong beer, the selected brewing equipment must contain the 11 kg of malt and still maintain efficiency on the system so that the mashing process is not impaired and the necessary sugar can be extracted.

The wort is prepared with malt (11 kg Pilsnermalt). The malt is then crushed and mixed with warm water (mash in) to reach an initial sugar content of SG 1097 which is measured with a hydrometer. Mash step profile is carried out (with a protein rest for mashes with unmodified grains or adjuncts) It is heated to 62.0 °C for 20 min, Saccharification. Then heated to 68.0 °C for 20 min (a-Amylase activity). Last step is heated to 72.0 °C in 20 min, then mash out at 78.0 °C for 1 min (sparge). The heat source of the pot is used to maintain the desired temperature during the mash.

Initial set-up: the wort is boiled for 75 minutes in total. The bittering hops Columbus (289 g) is added to the wort 45 minutes after start.

After boiling, the wort is transferred to a 30L fermenter, and fresh yeast (NCYC4260) is added. Then the fermenter is closed by water locks, and kept at a temperature of 9,4 C to start fermenting. After 12 days at 9.4 °C a sample can be taken for measuring the FG.

### Example 3d

### Fermentation of maltose at high Original Gravity (OG) conditions

### Fermentation profile see figure 8, bottom

Fermentation trials were carried out in 1 liter of wort (maltbased test brew).

The wort was prepared with pure organic malted barley extract (Barlex^{®} 7264 produced by Harboes Bryggeri A/S).

The organic malt extract was mixed with water to obtain a high maltsugar content to reach an initial sugar content of SG 1125 (specific gravity 1.125 gr/ml), measured with a hydrometer, and boiled shortly, then poured hot into a conical flask which was subsequently cooled to 15°C. 25 ml of the new yeast strain NCYC 4260 was added to the mixture of malt extract and water at a temperature of 15°C, and the initial OG of 1125 (specific gravity 1.125 gr/ml) was measured with a hydrometer. The fermentation mixture was placed at room temperature to start fermenting at 15-20°C. Then the conical flask was closed with a water lock and placed at room temperature for fermenting at 15-20°C. Fermentation started within 24 hours.

A sample was taken every day for measuring the SG. After 8 days the FG (final gravity) was 1037 (1.037 gr/ml). The measurement was repeated 3 times over 3 days to ensure fermentation had stopped.

The efficiency of the NCYC4260 yeast in the fermentation was 70,4%.

Alcohol level (final): 11,74 % vol.

### Example 3e

### Fermentation of maltose at low Original Gravity (OG) conditions

### Fermentation profile see figure 8, top

Fermentation trials were carried out in 1 liter of wort (maltbased test brew).

The wort was prepared with pure organic malted barley extract (Barlex^{®} 7264 produced by Harboes Bryggeri A/S).

The organic malt extract was mixed with water to obtain a high maltsugar content to reach an initial sugar content of SG 1011 (specific gravity 1.011 gr/ml), measured with a hydrometer, and boiled shortly, then poured hot into a conical flask which was subsequently cooled to 15°C. 25 ml of the new yeast strain NCYC 4260 was added to the mixture of malt extract and water at a temperature of 15°C, and the initial OG of 1011 (specific gravity 1.011 gr/ml) was measured with a hydrometer. The fermentation mixture was placed at room temperature to start fermenting at 15-20°C. Then the conical flask was closed with a water lock and placed at room temperature for fermenting at 15-20°C. Fermentation started within 24 hours.

A sample was taken every day for measuring the SG. After 5 days the FG (final gravity) was reached at 1001 (1.01 gr/ml). The measurement was repeated 3 times over 3 days to ensure fermentation had stopped.

The efficiency of the NCYC4260 yeast in the fermentation was 90,9%.

Alcohol level (final): 1,33 % vol.

### Example 3f

### Fermentation of apple juice

### Fermentation profile see figure 9, top

Fermentation trials were carried out in 1 liter of pure juice (fruit based brew from apples). The juice was a commercial, unfiltered pure press of juice made from apples, ('Vores Æble Juice' produced in Germany for Sailing Group).

The pure apple juice was carefully poured into a conical flask. 25 ml of the new yeast strain NCYC 4260 was added to the juice. The initial sugar content was measured with a hydrometer to have an SG of 1047 (specific gravity 1.047 gr/ml). Then the conical flask was closed with a water lock and placed at room temperature for fermenting 15-20°C. Fermentation started within 24 hours.

A sample was taken every day for measuring the SG. After 6 days the FG (final gravity) was reached at FG of 970 (0.97 gr/ml). The measurement was repeated 3 times over 3 days to ensure fermentation had stopped.

The efficiency of the NCYC4260 yeast in the fermentation was 106,4%.

Alcohol level (final): 10,27 % vol.

### Example 3g

### Fermentation tests of 5 basic sugars used in production of beer, wine and cider.

### Sucrose test

### Fermentation profile see figure 6, top

A mix of refined sucrose (table sugar) was mixed in a conical flask with boiling water starting with 80g to approximately 4.5x weight of water, and finally adjusted with water to an SG of 1060 (specific gravity 1.060 gr/ml). The solution was cooled to 15 °C and 25 ml of the yeast strain NCYC 4260 was added. The initial sugar content was measured with a hydrometer to an SG of 1060 (specific gravity 1.060 gr/ml). Then the conical flask was closed with a water lock and placed at room temperature for fermenting 15-20°C. Fermentation started within 24 hours.

A sample was taken every day for measuring the SG. After 12 days the FG (final gravity) was reached at 870 (0.87 gr/ml). The measurement was repeated 3 times over 3 days to ensure fermentation had stopped.

The efficiency (attenuation) of the NCYC4260 yeast in the fermentation was 78.3 %.

Alcohol level (final): 9.92 % vol.

### Fructose test

### Fermentation profile see figure 6, bottom

Pure fructose was mixed in a conical flask with boiling water starting with 80g to approximately 4,5 x weight of water, and finally adjusted with water to an SG of 1060 (specific gravity 1.060 gr/ml). The solution was cooled to 15 °C and 25 ml of the yeast strain NCYC 4260 was added. The initial sugar content was measured with a hydrometer to an SG of 1061 (specific gravity 1.061 gr/ml). Then the conical flask was closed with a water lock and placed at room temperature for fermenting 15-20°C. Fermentation started within 24 hours.

A sample was taken every day for measuring the SG. After 10 days the FG (final gravity) was reached at 860 (0.86 gr/ml) and measured min. 3 times over 3 days to ensure fermentation had stopped.

The efficiency (attenuation) of the NCYC4260 yeast in the fermentation was 77%.

Alcohol level (final): 10 % vol.

### Glucose test

### Fermentation profile see figure 7, top

Glucose syrup (Pure Glucose syrup, Morten Heiberg.dk), 55g was mixed in a conical flask with 220g of boiling water and finally adjusted with water to an SG of 1060 (specific gravity 1.060 gr/ml). The solution was cooled to 15°C and 25 ml of the yeast strain NCYC 4260 was added. The initial sugar content was measured with a hydrometer to an SG of 1059 (specific gravity 1.059 gr/ml). Then the conical flask was closed with a water lock and placed at room temperature for fermenting 15-20°C. Fermentation started within 24 hours.

A sample was taken every day for measuring the SG. After 7 days the FG (final gravity) was reached at 1037 (1.037 gr/ml) and measured 3 times over 3 days to ensure fermentation had stopped.

The efficiency (attenuation) of the NCYC4260 yeast in the fermentation was 2,1%.

Alcohol level (final): 2,93 % vol.

### Lactose test

### Fermentation profile see figure 7, midle

Lactose and boiling water was mixed and SG was measured with a hydrometer to be 1022, then cooled to 15°C. 25 ml of the yeast strain NCYC 4260 was added and the SG was measured again with a hydrometer to be 1021(specific gravity 1.021 gr/ml).

The SG was measured daily for 4 days and was found after 4 days to be identical to the start value, *i.e.* FG = 1021. The fermentation test was accordingly stopped.

The efficiency/attenuation of the NCYC4260 yeast in the fermentation was 0.

Alcohol level (final): 0, no fermentation had taken place

### Maltodextrin test

### Fermentation profile see figure 7, bottom

Maltodextrin and boiling water was mixed and SG was measured with a hydrometer to be 1023, then cooled to 15°C. 25 ml of the yeast strain NCYC 4260 was added and the SG was measured again with a hydrometer to be 1023 (specific gravity 1.023 gr/ml).

The SG was measured daily for 4 days and was found after 4 days to be identical to the start value, *i.e.* FG = 1023. The fermentation test was accordingly stopped.

The efficiency/attenuation of the NCYC4260 yeast in the fermentation was 0.

Alcohol level (final): 0, no fermentation had taken place

### Example 4 Tasting notes

The two beers Test 1 and Test 2 brewed according to Example 3 hereinabove were tasted by a panel of two tasting-experts who had no knowledge of the brewing process, yeast type employed or alcohol strength of the tasted beers.

The impression was that both beers had a very clean taste with a delightful malty and good body. The specific comments from the experts were:
- **Test 1:** tastes clean and malty, caramel notes, perceived ABV 5-6 % (actual ABV = 5%)
- **Test 2:** tastes clean and malty, perceived ABV 5-6 % (actual ABV = 2,8%)

The tasting experts surprisingly perceived the **Test 2** lager of 2,8% ABV to have a similar alcohol content as the normal-strength **Test 1** lager of 5,0%. Without wanting to be bound by theory, the inventors believe that the impression or perception of alcohol strength is linked closely to the body, texture and mouthfeel of the beer, and that these factors are again closely related to the composition of residual sugars and carbohydrates in the beer when the fermentation is over. For the new yeast strain NCYC 4260 the fermentation takes significantly longer and the attenuation is lower than by using the traditional lager yeast *S.pastorianus,* for example. It is not yet known exactly which residual sugars and other carbohydrates are left by NCYC 4260, but it seems clear that NCYC 4260 has an ability to produce a lager having a body which gives an impression of a higher alcohol content than it actually contains.

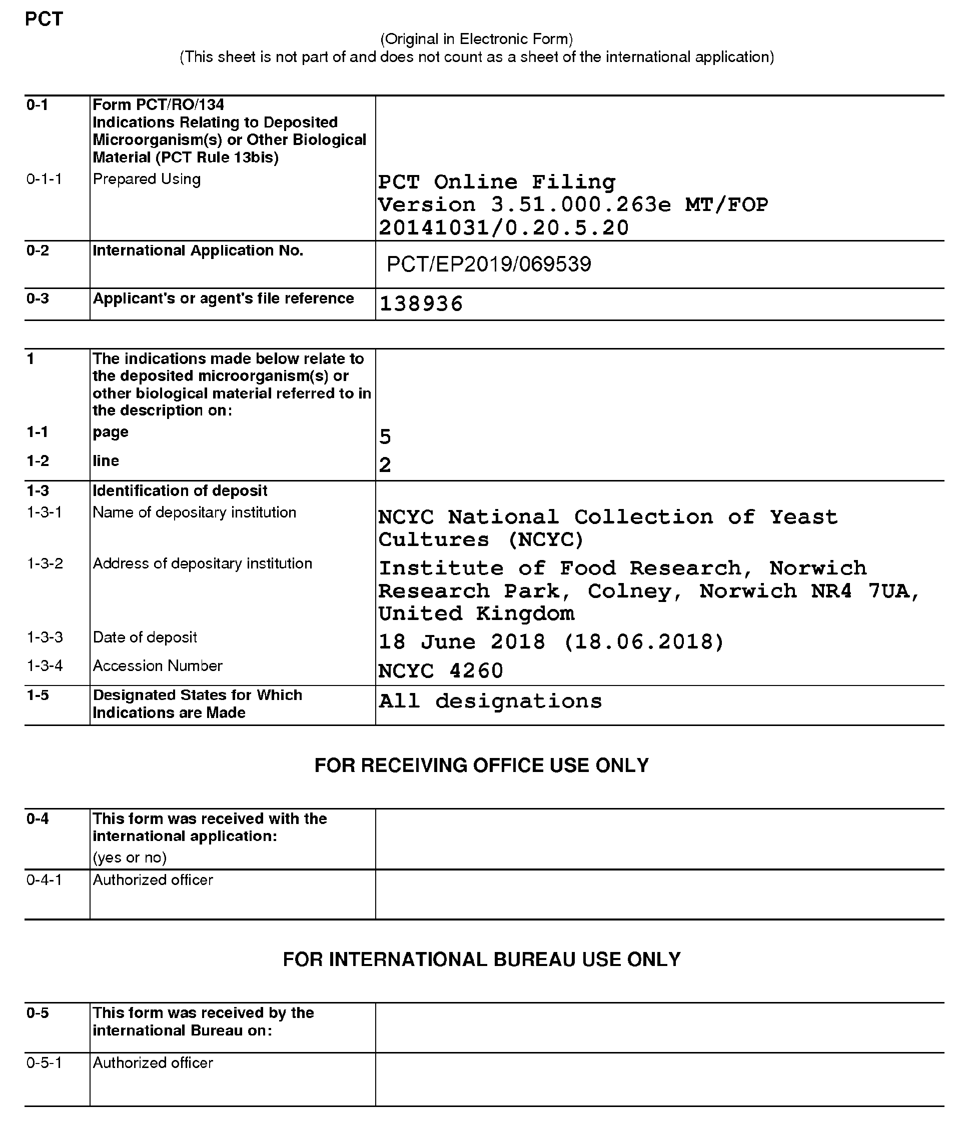

## Claims

1. Yeast strain belonging to the species *Saccharomyces cerevisiae* having the identification code NCYC 4260.

2. A biologically pure culture of a yeast strain deposited as NCYC 4260 or subcultures thereof.

3. A yeast cell culture comprising yeast cells from the yeast strain deposited as NCYC 4260.

4. A method for preparing a yeast cell culture, said method comprising:
(a) propagating a yeast cell from the strain of claim 1 or from the culture of 2; and
(b) obtaining the yeast cell culture from step (a).

5. Use of the yeast strain of claim 1 or the culture of claims 2 for the fermentation of worts and other sources of fermentable sugars in the preparation of an alcoholic beverage, wherein the fermentation is carried out at a temperature between 3-15 °C, such as 3-10 °C or such as 6-10 °C.

6. The use of the yeast strain according to claim 5 for the fermentation of worts and other sources of fermentable sugars, wherein the resulting alcoholic beverage is beer, sake, wine, cider, mead, root-beer, ginger-beer and kampuchea.

7. The use of the yeast strain according to claim 5 or 6, wherein the resulting alcoholic beverage is apple cider.

8. A method of preparing beer, comprising the steps of:
a) providing a wort; and
b) fermenting the wort with a yeast strain of claim 1 or the culture of of claims 2 to obtain a beer under sterile conditions and with the yeast strain of claim 1 or the culture of claims 2 as the only species of yeast added; and
c) optionally pasteurizing said beer obtained under b) to remove live yeast cells; and
d) optionally subjecting said beer to a period of cold storage (lagering).

9. The method according to claim 8, wherein the fermentation of the wort with the yeast strain of claim 1 or the culture of claims 2 is carried out for at least 12 days.

10. The method according to any one of claims 8 to 9, wherein the fermentation of the wort with the yeast strain of claim 1 or the culture of claims 2 is carried out at a temperature between 3-10 °C, such as 6-10 °C.

11. The method of any one of claim 8-10, wherein the beer is a lager beer.

12. The method according to any one of claims 8 to 11, wherein the fermentation of the wort with the yeast strain of claim 1 or the culture of claims 2 provides a beer with an alcohol content of between 0.05 and 0.5% (vol/vol).

13. The method according to any one of claims 8 to 11, wherein the fermentation of the wort with the yeast strain of claim 1 or the culture of claims 2 provides a beer with an alcohol content of between 0.5 and 2.8% (vol/vol) and a final gravity (FG) of between 1001 and 1025, such as 1010.

14. The method according to any one of claims 8 to 11, wherein the fermentation of the wort with the yeast strain of claim 1 or the culture of claims 2 provides a beer with an alcohol content of between 2.8 and 14% (vol/vol) and a final gravity (FG) of between 1001 and 1037, such as 1010.

15. A beer obtainable by a method according to any one of claims 8-14 which comprises live yeast cells identical to yeast cells from the yeast strain deposited as NCYC 4260 or subcultures thereof.

## Patentansprüche

1. Zur Spezies *Saccharomyces cerevisiae* gehörender Hefestamm mit dem Identifikationscode NCYC 4260.

2. Biologisch reine Kultur eines als NCYC 4260 hinterlegten Hefestamms oder Subkulturen davon.

3. Hefezellkultur, die Hefezellen des als NCYC 4260 hinterlegten Hefestamms umfasst.

4. Verfahren zur Herstellung einer Hefezellkultur, wobei das Verfahren Folgendes umfasst:
(a) das Vermehren einer Hefezelle des Stamms von Anspruch 1 oder der Kultur von Anspruch 2 und
(b) den Erhalt der Hefezellkultur von Schritt (a).

5. Verwendung des Hefestamms nach Anspruch 1 oder der Kultur nach Anspruch 2 zur Fermentation von Bierwürzen und anderen Quellen für fermentierbare Zucker bei der Herstellung eines alkoholischen Getränks, wobei die Fermentation bei einer Temperatur zwischen 3-15 °C, wie 3-10 °C oder wie 6-10 °C, durchgeführt wird.

6. Verwendung des Hefestamms nach Anspruch 5 zur Fermentation von Bierhefen und anderen Quellen für fermentierbare Zucker, wobei es sich beim resultierenden alkoholischen Getränk um Bier, Sake, Wein, Cider, Met, Root Beer, Ingwerbier und Kombucha handelt.

7. Verwendung des Hefestamms nach Anspruch 5 oder 6, wobei es sich beim resultierenden alkoholischen Getränk um Apfelwein handelt.

8. Verfahren zur Herstellung von Bier, das die folgenden Schritte umfasst:
a) das Bereitstellen einer Bierwürze und
b) das Fermentieren der Bierwürze mit einem Hefestamm nach Anspruch 1 oder der Kultur nach Anspruch 2, wodurch ein Bier unter sterilen Bedingungen erhalten wird, und mit dem Hefestamm nach Anspruch 1 oder der Kultur nach Anspruch 2 als den einzigen Spezies von zugegebener Hefe; und
c) gegebenenfalls das Pasteurisieren des in b) erhaltenen Biers, wodurch lebende Hefezellen entfernt werden; und
d) gegebenenfalls das Einwirkenlassen eines Zeitraums einer Kaltlagerung (Reifung) auf das Bier.

9. Verfahren nach Anspruch 8, wobei die Fermentation der Bierwürze mit dem Hefestamm nach Anspruch 1 oder der Kultur nach Anspruch 2 mindestens 12 Tage lang erfolgt.

10. Verfahren nach einem der Ansprüche 8 bis 9, wobei die Fermentation der Bierwürze mit dem Hefestamm nach Anspruch 1 oder der Kultur nach Anspruch 2 bei einer Temperatur zwischen 3-10 °C, wie 6-10 °C, erfolgt.

11. Verfahren nach einem der Ansprüche 8-10, wobei es sich beim Bier um ein untergäriges Bier handelt.

12. Verfahren nach einem der Ansprüche 8 bis 11, wobei die Fermentation der Bierwürze mit dem Hefestamm nach Anspruch 1 oder der Kultur nach Anspruch 2 ein Bier mit einem Alkoholgehalt zwischen 0,05 und 0,5 Vol.-% ergibt.

13. Verfahren nach einem der Ansprüche 8 bis 11, wobei die Fermentation der Bierwürze mit dem Hefestamm nach Anspruch 1 oder der Kultur nach Anspruch 2 ein Bier mit einem Alkoholgehalt zwischen 0,5 und 2,8 Vol.-% und einer Enddichte (FG) zwischen 1001 und 1025, wie 1010, ergibt.

14. Verfahren nach einem der Ansprüche 8 bis 11, wobei die Fermentation der Bierwürze mit dem Hefestamm nach Anspruch 1 oder der Kultur nach Anspruch 2 ein Bier mit einem Alkoholgehalt zwischen 2,8 und 14 Vol.-% und einer Enddichte (FG) zwischen 1001 und 1037, wie 1010, ergibt.

15. Bier, das durch ein Verfahren nach einem der Ansprüche 8-14 erhältlich ist, das lebende Hefezellen umfasst, die mit Hefezellen aus dem als NCYC 4260 hinterlegten Hefestamm oder Subkulturen davon identisch sind.

## Revendications

1. Souche de levure appartenant à l'espèce *Saccharomyces cerevisiae* ayant le code d'identification NCYC 4260.

2. Culture biologiquement pure d'une souche de levure déposée en tant que NCYC 4260 ou de sous-cultures de celle-ci.

3. Culture de cellules de levure comprenant des cellules de levure provenant de la souche de levure déposée en tant que NCYC 4260.

4. Procédé pour la préparation d'une culture de cellules de levure, ledit procédé comprenant :
(a) la propagation d'une cellule de levure provenant de la souche selon la revendication 1 ou provenant de la culture selon la revendication 2 ; et
(b) l'obtention de la culture de cellules de levure provenant de l'étape (a).

5. Utilisation de la souche de levure selon la revendication 1 ou de la culture selon la revendication 2 pour la fermentation de moûts et d'autres sources de sucres fermentescibles dans la préparation d'une boisson alcoolisée, dans laquelle la fermentation est effectuée à une température comprise entre 3 et 15 °C, telle qu'entre 3 et 10 °C ou telle qu'entre 6 et 10 °C.

6. Utilisation de la souche de levure selon la revendication 5 pour la fermentation de moûts et d'autres sources de sucres fermentescibles, dans laquelle la boisson alcoolisée résultante est de la bière, du saké, du vin, du cidre, de l'hydromel, de la root beer, de la ginger beer et du kombucha.

7. Utilisation de la souche de levure selon la revendication 5 ou 6, dans laquelle la boisson alcoolisée résultante est du cidre de pomme.

8. Procédé de préparation de bière, comprenant les étapes consistant à :
a) fournir un moût ; et
b) fermenter le moût avec une souche de levure selon la revendication 1 ou la culture selon la revendication 2 pour obtenir une bière dans des conditions stériles et avec la souche de levure selon la revendication 1 ou la culture selon la revendication 2 en tant qu'unique espèce de levure ajoutée ; et
c) éventuellement pasteuriser ladite bière obtenue en
b) pour éliminer les cellules de levure vivantes ; et
d) éventuellement soumettre ladite bière à une période de conservation à froid (garde).

9. Procédé selon la revendication 8, dans lequel la fermentation du moût avec la souche de levure selon la revendication 1 ou la culture selon la revendication 2 est effectuée pendant au moins 12 jours.

10. Procédé selon l'une quelconque des revendications 8 à 9, dans lequel la fermentation du moût avec la souche de levure selon la revendication 1 ou la culture selon la revendication 2 est effectuée à une température comprise entre 3 et 10 °C, telle qu'entre 6 et 10 °C.

11. Procédé selon l'une quelconque des revendications 8 à 10, dans lequel la bière est une bière blonde.

12. Procédé selon l'une quelconque des revendications 8 à 11, dans lequel la fermentation du moût avec la souche de levure selon la revendication 1 ou la culture selon la revendication 2 fournit une bière présentant une teneur en alcool comprise entre 0,05 et 0,5 % (v/v).

13. Procédé selon l'une quelconque des revendications 8 à 11, dans lequel la fermentation du moût avec la souche de levure selon la revendication 1 ou la culture selon la revendication 2 fournit une bière présentant une teneur en alcool comprise entre 0,5 et 2,8 % (v/v) et une densité finale (DF) comprise entre 1001 et 1025, telle que de 1010.

14. Procédé selon l'une quelconque des revendications 8 à 11, dans lequel la fermentation du moût avec la souche de levure selon la revendication 1 ou la culture selon la revendication 2 fournit une bière présentant une teneur en alcool comprise entre 2,8 et 14 % (v/v) et une densité finale (DF) comprise entre 1001 et 1037, telle que de 1010.

15. Bière pouvant être obtenue par un procédé selon l'une quelconque des revendications 8 à 14 qui comprend des cellules de levure vivantes identiques à des cellules de levure provenant de la souche de levure déposée en tant que NCYC 4260 ou de sous-cultures de celle-ci.
